Europäisches Patentamt
European Patent Office
Office européen des brevets

(19) (11) Veröffentlichungsnummer: 0 176 875 A2

# (12) EUROPÄISCHE PATENTANMELDUNG

(21) Anmeldenummer: 85111834.9

(22) Anmeldetag: 19.09.85

(51) Int. Cl.[4]: C 07 D 257/04
A 61 K 31/41

(30) Priorität: 22.09.84 DE 3434948

(43) Veröffentlichungstag der Anmeldung: 09.04.86 Patentblatt 86/15

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Frickel, Fritz-Frieder, Dr.
Silvanerweg 7
D-6705 Deidesheim(DE)

(72) Erfinder: Nuerrenbach, Axel, Dr.
Koenigsberger Strasse 7
D-6718 Gruenstadt(DE)

(72) Erfinder: Thyes, Marco, Dr.
Thorwaldsenstrasse 1
D-6700 Ludwigshafen(DE)

(54) **Vinyltetrazolylphenyl-Derivate, ihre Herstellung und Verwendung.**

(57) Es werden 1,3- und 1,4-substituierte Vinyltetrazolylphenyl-Derivative der Formel (I)

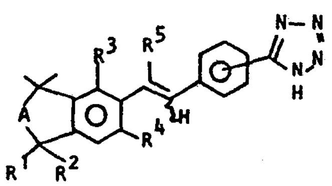

beschrieben, worin A, $R^1$, $R^2$, $R^3$, $R^4$ und $R^5$ die angegebene Bedeutung haben. Die neuen Verbindungen stellen Arzneimittelwirkstoffe dar.

EP 0 176 875 A2

Vinyltetrazolylphenyl-Derivate, ihre Herstellung und Verwendung

Die vorliegende Erfindung betrifft neue substituierte Vinyltetrazolphenyl--Derivate, deren Herstellung, Arzneimittel, die diese Verbindungen enthalten, sowie deren Verwendung bei der Bekämpfung von Krankheiten.

Es ist beispielsweise aus den DE-OS 28 54 354 und 32 02 118 bekannt, daß Vinylbenzoesäure-Derivate pharmakologische Wirkungen bei der topischen und systemischen Therapie von Neoplasien, Akne, Psoriasis und anderen dermatologischen Affektionen aufweisen. Diese Vinylbenzoesäure-Derivate befriedigen jedoch nicht immer. Nachteilig ist vor allen Dingen ihre ausgeprägte toxische (Neben-)Wirkung, die diese Verbindungen als Mittel in der topischen und systemischen Therapie von Neoplasien, Akne, Psoriasis und anderen dermatologischen Affektionen wenig geeignet erscheinen lassen. Die Nachteiligkeit der Verbindungen der DE-OS 28 54 354 wird beispielsweise von A. Kistler in Calcified Tissue International 33, 249-254 (1981) beschrieben und offenbart sich bei mehrmaliger Applikation an Nagern, nach der von R. C. Moon et al in Cancer Research 39, 1339-1346 (1979) beschriebenen Methodik.

Der vorliegenden Erfindung liegt die Aufgabe zugrunde, Verbindungen mit vergleichbarer Wirkungsstärke und geringeren toxischen Nebenwirkungen zur Verfügung zu stellen.

Es wurde gefunden, daß sich Vinyltetrazolylphenyl-Derivate der Formel (I)

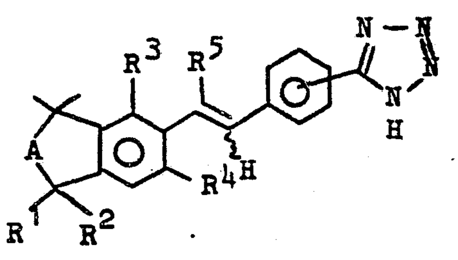 (I),

in der $R^1$ und $R^2$ Wasserstoff oder eine Methylgruppe,
$R^3$ und $R^4$ Wasserstoff, Halogen, eine $C_{1-4}$-Alkyl- oder $C_{1-4}$-Alkoxygruppe,
$R^5$ Wasserstoff, eine $C_{1-6}$-Alkyl- oder eine $C_{3-6}$-Cycloalkylgruppe und
A einen gegebenenfalls durch $C_{1-4}$-Alkylgruppen substituierten Methylen- oder Ethylenrest oder - wenn $R^1$ und $R^2$ Methylgruppen darstellen - auch die Reste $-CH_2-CO-$ oder $-CH_2-CH(OH)-$ bedeuten, und ihre physiologisch verträglichen Salze gut zur Behandlung von Krankheiten eignen.

Wie die wellenförmig dargestellte C-H-Bindung in der Formel I angibt, können die erfindungsgemäßen Verbindungen in der cis-(Z)- oder trans-(E)--Form vorliegen. Die trans-E-Formen sind bevorzugt. Als bevorzugte Alkylgruppe an A ist die Methylgruppe zu nennen. Für $R^3$ und $R^4$ sind Wasserstoff, Methyl und Methoxy bevorzugt. $R^5$ ist vorzugsweise Wasserstoff oder Methyl.

Typische Beispiele für erfindungsgemäße Verbindungen sind außer den in den Beispielen genannten Substanzen:

2-(1,1,2,3,3-Pentamethyl-2,3-dihydro-5(1H)-indenyl)-1-[4-(1H-tetrazol--5-yl)phenyl]-ethen

2-(1,1,3,3-Tetramethyl-2,3-dihydro-5(1H)-indenyl)-1-[4-(1H-tetrazol-5--yl)phenyl]-ethen

2-(1,1,4,4-Tetramethyl-1,2,3,4-tetrahydronaphth-6-yl)-1-[4-(1H-tetrazol--5-yl)phenyl]-1-buten

2-(1,1,3,3-Tetramethyl-2,3-dihydro-5(1H)-indenyl)-1-[4-(1H-tetrazol-5--yl)phenyl]-1-buten

2-(1,1,2,3,3-Pentamethyl-2,3-dihydro-5(1H)-indenyl)-1-[4-(1H-tetrazol--5-yl)phenyl]-1-buten

2-(1,1,4,4-Tetramethyl-1,2,3,4-tetrahydronaphth-6-yl)-1-[4-(1H-tetrazol--5-yl)phenyl]-1-octen

2-(1,1,3,3-Tetramethyl-2,3-dihydro-5(1H)-indenyl)-1-[4-(1H-tetrazol-5--yl)phenyl]-1-octen

2-(1,1,2,3,3-Pentamethyl-2,3-dihydro-5(1H)-indenyl)-1-[4-(1H-tetrazol--5-yl)phenyl]-1-octen

2-(1,1,4,4,7-Pentamethyl-1,2,3,4-tetrahydronaphth-6-yl)-1-[4-(1H-tetrazol-5-yl)phenyl]-1-propen

2-(1,1,3,3,6-Pentamethyl-2,3-dihydro-5(1H)-indenyl)-1-[4-(1H-tetrazol--5-yl)phenyl]-1-propen

2-(1,1,2,3,3,6-Hexamethyl-2,3-dihydro-5(1H)-indenyl)-1-[4-(1H-tetrazol--5-yl)phenyl]-1-propen

2-(7-Ethyl-1,1,4,4-Tetramethyl-1,2,3,4-tetrahydronaphth-6-yl)-1-[4-(1H--tetrazol-5-yl)phenyl]-1-propen

2-(6-Ethyl-1,1,3,3-tetramethyl-2,3-dihydro-5(1H)-indenyl)-1-[4-(1H-tetrazol-5-yl)phenyl]-1-propen

2-(6-Ethyl-1,1,2,3,3-pentamethyl-2,3-dihydro-5(1H)-indenyl)-1-[4-(1H--tetrazol-5-yl)phenyl]-1-propen

2-[7-(2)-Methylethyl)-1,1,4,4-tetramethyl-1,2,3,4-tetrahydronaphth-6--yl]-1-[4-(1H-tetrazol-5-yl)phenyl]-1-propen

2-[6-(2-Methylethyl)-1,1,2,3,3-pentamethyl-2,3-dihydro-5(1H)-indenyl)--1-[4-(1H-tetrazol-5-yl)phenyl]-1-propen

2-[6-(2-Methylethyl)-1,1,3,3-tetramethyl-2,3-dihydro-5(1H)-indenyl)-1--[4-(1H-tetrazol-5-yl)phenyl]-1-propen

2-(1,1,4,4-Tetramethyl-1,2,3,4-tetrahydronaphth-6-yl)-1-[3-1H-tetrazol--5-yl)phenyl]-1-propen

2-(1,1,3,3-Tetramethyl-2,3-dihydro-5(1H)-indenyl)-1-[3-(1H-tetrazol-5--yl)phenyl]-1-propen

2-(1,1,2,3,3-Pentamethyl-2,3-dihydro-5(1H)-indenyl)-1-[3-(1H-tetrazol--5-yl)phenyl]-1-propen

2-(7-butyl-1,1,4,4-tetramethyl-1,2,3,4-tetrahydronaphth-6-yl)-1-[4-(1H--tetrazol-5-yl)phenyl]-1-propen

2-(6-Butyl-1,1,3,3-tetramethyl-2,3-dihydro-5(1H-indenyl)-1-[4-(1H-tetrazol-5-yl)phenyl]-1-propen

2-(6-Butyl-1,1,2,3,3-pentamethyl-2,3-dihydro-5(1H)-indenyl)-1-[4-(1H--tetrazol-5-yl)phenyl]-1-propen

2-(1,1,4,4-Tetramethyl-1,2,3,4-tetrahydronaphth-6-yl)-2-cyclopropyl-1--[4-(1H-tetrazol-5-yl)phenyl]ethen

2-(1,1,3,3-Tetramethyl-2,3-dihydro-5(1H)-indenyl)-2-cyclopropyl-1-[4-(1H--tetrazol-5-yl)phenyl]-ethen

2-(1,1,2,3,3-Pentamethyl-2,3-dihydro-5(1H)-indenyl)-2-cyclopropyl-1-[4--tetrazol-5-yl)phenyl]-ethen

2-(1,1,2,3,3,6-Hexamethyl-2,3-dihydro-5(1H)-indenyl)-2-cyclopropyl-1-[4-(1H-tetrazol-5-yl)phenyl]-ethen

2-(1,1,3,3,6-Pentamethyl-2,3-dihydro-5(1H)-indenyl)-2-cyclopropyl-1-[4-(1H-tetrazol-5-yl)phenyl]-1-[4-(1H-tetrazol-5-yl)phenyl]-ethen

2-(1,1,3,3,7-Pentamethyl-1,2,3,4-tetrahydronaphth-6-yl)-2-cyclopropyl-1-[4-(1H-tetrazol-5-yl)phenyl]-1-[4-(1H-tetrazol-5-yl)phenyl]-ethen

2-(1,1,4,4-Tetramethyl-1,2,3,4-tetrahydronaphth-6-yl)-1-[4-(1H-tetrazol-5-yl)phenyl]-(3-methyl)-buten-1

2-(1,1,3,3-Tetramethyl-2,3-dihydro-5(1H)-indenyl)-1-[4-(1H-tetrazol-5-yl)phenyl]-(3-methyl)-buten-1

2-(1,1,2,3,3-Pentamethyl-2,3-dihydro-5(1H)-indenyl)-1-[4-(1H-tetrazol-5-yl)phenyl]-(3-methyl)-buten-1

2-(1,1,3,3-Tetramethyl-2,3-dihydro-5(1H)-indenyl)-2-cyclohexyl-1-[4-(1H-tetrazol-5-yl)phenyl]-ethen

2-(1,1,2,,3,3-Pentamethyl-2,3-dihydro-5(1H)-indenyl)-2-cyclohexyl-1-[4-(1H-tetrazol-5-yl)phenyl]-ethen

2-(1,1,4,4-Tetramethyl-1,2,3,4-tetrahydronaphth-6-yl)-2-cyclohexyl-1-[4-(1H-tetrazol-5-yl)phenyl]-ethen

2-(4,4,7-Trimethyl-1,2,3,4-tetrahydronaphth-6-yl)-1-[4-(1H-tetrazol-5-yl)phenyl]-1-propen

2-(4,4-Dimethyl-1,2,3,4-tetrahydronaphth-6-yl)-1-[4-(1H-tetrazol-5-yl)phenyl]-1-propen

2-(1,1,4,4-Tetramethyl-1,2,3,4-tetrahydro-2-oxo-naphth-6-yl)-1-[4-(1H-tetrazol-5-yl)phenyl]-1-propen

2-(1,1,4,4-Tetramethyl-1,2,3,4-tetrahydro-2,3-dioxo-naphth-6-yl)-1-[4-(1H-tetrazol-5-yl)phenyl]-1-propen

2-(1,1,3,3-Tetramethyl-2,3-dihydro-5(1H)-2-oxo-indenyl)-1-[4-(1H-tetrazol-5-yl)phenyl]-1-propen

2-(1,1,4,4-Tetramethyl-1,2,3,4-tetrahydro-3-oxo-naphth-6-yl)-1-[4-(1H--tetrazol-5-yl)phenyl]-1-propen

2-(1,1,3,3-Tetramethyl-2,3-dihydro-5(1H)-2-hydroxy-indenyl)-1-[4-(1H--tetrazol-5-yl)phenyl]-1-propen

2-(1,1,4,4-Tetramethyl-1,2,3,4-tetrahydro-2-hydroxy-naphth-6yl)-1-[4-(1H--tetrazol-5-yl)phenyl]-1-propen

2-(1,1,4,4-Tetramethyl-1,2,3,4-tetrahydro-3-hydroxy-naphth-6-yl)-1-[4--(1H-tetrazol-5-yl)phenyl]-1-propen

2-(1,1,4,4-Tetramethyl-1,2,3,4-tetrahydro-2-oxo-naphth-6-yl)-1-[4-(1H--tetrazol-5-yl)phenyl]-ethen

2-(1,1,4,4-Tetramethyl-1,2,3,4-tetrahydro-3-oxo-naphth-6-yl)-1-[4-(1H--tetrazol-5-yl)phenyl]-ethen

2-(1,1,4,4-Tetraethyl-1,2,3,4-tetrahydro-2-oxo-naphth-6-yl)-1-[4-(1H-tetrazol-5-yl)phenyl]-1-buten

2-(1,1,4,4-Tetramethyl-1,2,3,4-tetrahydro-3-oxo-naphth-6-yl)-1-[4-(1H--tetrazol-5-yl)phenyl]-1-buten

Die erfindungsgemäßen Verbindungen werden hergestellt durch Umsetzen eines Azids mit einem Nitril der Formel (II)

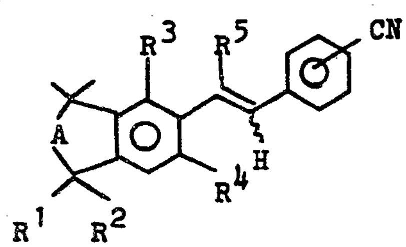

(II),

in der $R^1$, $R^2$, $R^3$, $R^4$, $R^5$ und A die oben angegebenen Bedeutungen haben.

Die Umsetzung mit dem Azid unter Tetrazolbildung erfolgt bei einer Temperatur von etwa Raumtemperatur bis zu etwa 200°C, vorzugsweise bei 60 bis 100°C. Die Umsetzung kann bei Normaldruck oder in geschlossenen Gefäßen unter erhöhtem Druck zweckmäßig unter Erwärmung auf den angegebenen Temperaturbereich durchgeführt werden.

Zweckmäßigerweise wird die Umsetzung in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines Alkylformamids, wie Dimethyl- oder Diethylformamid, in protischen Lösungsmitteln wie Alkoholen oder in Mischungen der genannten Lösungsmittel durchgeführt. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur von 60 bis 100°C abläuft.

Es empfiehlt sich, die Umsetzung in Gegenwart eines anorganischen Halogenids vorzunehmen. Als anorganische Halogenide kommen insbesondere in Betracht: Ammoniumchlorid, Lithiumchlorid, Aluminium(III)chlorid.

Als Azide kommen vorzugsweise Alkaliazide, wie Lithiumazid, Natriumazid oder Kaliumazid, vorzugsweise Natriumazid oder 1,1,3,3-Tetramethylguanidiniumazid in Betracht.

Die Ausgangsverbindungen der Formel (II) können einheitlich die cis- oder trans-Struktur haben oder Mischungen der beiden E-/Z-Isomeren darstellen. Eine Isomerisierung bei der Herstellung der neuen Verbindungen in sterisch einheitlicher Form kann nicht ausgeschlossen werden. Die in solchen Fällen resultierende Isomeren-Mischung der erfindungsgemäßen Verbindungen der Formel (I) kann durch HPLC-Analyse oder durch ein $^{13}C$-NMR-Spektrum quantitativ bestimmt und das jeweils gewünschte Isomere gegebenenfalls durch fraktionierende Kristallisation oder Chromatographie mit beispielsweise Kieselgelsäulen oder durch präparative HPL-Chromatographie isomerenrein isoliert werden.

Die Herstellung der Ausgangsnitrile der Formel (II) ist in der DE-OS 32 02 125 beschrieben oder kann gemäß der dort angegebenen Arbeitsvorschrift erfolgen. Beispielsweise kann eine Carbonylverbindung der Formel (III)

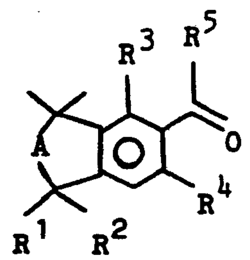 (III),

in der $R^1$, $R^2$, $R^3$, $R^4$, $R^5$ und A die oben angegebenen Bedeutungen haben mit einer para- oder meta-substituierten Phosphorverbindung der Formel (IV)

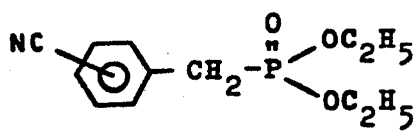 (IV),

in einer Horner-Emmons-Reaktion umgesetzt werden.

Die erfindungsgemäßen Verbindungen weisen ein acides Wasserstoffatom am Tetrazolring auf und können daher mit Basen in üblicher Weise in ein physiologisch verträgliches, wasserlösliches Salz überführt werden. Geeignete Salze sind beispielsweise Ammonium-, Alkalimetall- (insbesondere des Natriums, Kaliums und Lithiums) und Erdalkalimetallsalze, insbesondere des Calciums oder Magnesiums sowie Salze mit geeigneten organischen Basen, wie mit niederen Alkylaminen, z.B. Methylamin oder Ethylamin, mit substituierten niederen Alkylaminen, insbesondere hydroxysubstituierten Alkylaminen, wie Diethanolamin, Triethanolamin oder Tris-hydroxymethyl--aminomethan, Piperidin oder Morpholin.

Die erfindungsgemäßen Verbindungen und ihre physiologisch verträglichen Salze können aufgrund ihrer pharmakologischen Eigenschaften bei der topischen und systemischen Therapie und auch Prophylaxe von Präkanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Bevorzugte Indikationsgebiete sind neben der Therapie von dermatologischen Erkrankungen die prophylaktische und therapeutische Behandlung von Präkanzerosen und Tumoren sowie die Behandlung arthritischer Erkrankungen. Die dermatologische Aktivität, beispielsweise für

die Behandlung von Akne, kann u. a. durch die Bestimmung der komedolytischen Aktivität und die Fähigkeit nachgewiesen werden, die Anzahl der Utrikuli im Modell der Rhino-Maus zu reduzieren. Diese Methode ist von L.H. Kligman et al. in The Journal of Investigative Dermatology 73, 354 bis 38 (1979) und von J.A. Mezick et al. in "Models of Dermatology (Ed. Maibach, Lowe) Vol. 2, S. 59-63, Karger, Basch 1985 beschrieben. Die antiarthritische Wirkung der erfindungsgemäßen Verbindungen kann in üblicher Weise im Tierexperiment im Adjuvans-Arthritis-Modell bestimmt werden. Die präventive Wirkung bei der Entstehung und Entwicklung prämaligner Läsionen können beispielsweise in den nachfolgenden Testmodellen aufgezeigt werden. Die erfindungsgemäßen Verbindungen heben an Hamstertreachealgewebe in vitro die nach Vitamin-A-Mangel einsetzende Keratinisierung auf. Diese Keratinisierung gehört zur Frühphase der Carcinogenese, die in einer ähnlichen Technik in vivo nach der Initiation durch chemische Verbindungen, durch energiereiche Strahlung oder nach viraler Zelltransformation durch die erfindungsgemäßen Verbindungen der Formel (I) inhibiert wird. Diese Methodik kann aus Cancer Res. 36, 964 bis 972 (1976) oder aus Nature 250, 64 bis 66 (1974) und Nature 253, 47 bis 50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035 bis 1041 (1978), Experimental Cell Research 117, 15 bis 22 (1978) und Proc. Natl. Acad. Sci. USA 77, 2936 bis 2940 (1980) entnommen werden. Weiterhin sei auf die Bestimmung der antagonistischen Wirkungen gegenüber Phorbolester verwiesen, die in Cancer Res. 37, 2196 bis 2201 (1977) beschrieben wird.

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Bekämpfung von Krankheiten.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung vorzugsweise in einer Einzeldosis von 0,1 bis 50 mg enthalten und täglich in einer oder mehrerer Dosierungen je nach Art und Schwere der Erkrankung verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxyltoluol oder geschmacksverbessernde Zusätze, Stabilisierungsmittel, Emulgiermittel, Gleitmittel usw. zugesetzt werden. Voraussetzung ist, daß alle bei der Herstellung pharmazeutischer Zubereitungen verwendeten Stoffe toxikologisch unbedenklich sind und mit den verwendeten Wirkstoffen verträglich sind.

Herstellung der erfindungsgemäßen Verbindungen

Beispiel 1

(E)-2-(1,1,4,4-Tetramethyl-1,2,3,4-tetrahydronaphth-6-yl)-1-[4-(1H-tetrazol-5-yl)phenyl]-1-propen

Zu einer Suspension von 65 g Natriumazid in 500 ml Tetrahydrofuran wurden bei 0°C in kleinen Portionen 33,5 g wasserfreies Aluminiumchlorid eingetragen. Danach wurde ca. 45 min unter Rückfluß erhitzt, mit 16,5 g (E)-4-[2-(5,6,7,8-Tetrahydro-5,5,8,8-tetramethyl-2-naphthalenyl)-1-propenyl]benzonitril versetzt und anschließend nochmals 24 h bei Rückflußtemperatur gerührt. Nach dem Erkalten wurde das Reaktionsgemisch auf 2,5 l Eiswasser gegossen, mit 250 ml Ethanol versetzt und mit 2 N Salzsäure angesäuert. Der entstandene Feststoff wurde abfiltriert und auf dem Filter mit 100 ml kaltem Methanol gewaschen. Nach dem Trocknen im Stickstoffstrom verblieben 12,2 g (66 % d.Th.) (E)-2-(1,1,4,4-Tetramethyl-1,2,3,4-tetrahydronaphth-6-yl)-1-[4-(1H-tetrazol-5-yl)phenyl]-1-propen, Fp. 227 bis 230°C. Das $^{13}C$-NMR-Spektrum belegt, daß es sich um die isomerenreine trans-konfigurierte Titelverbindung handelt.
Analog wurden die Substanzen der nachfolgenden Tabelle hergestellt.

| Nr. | $R^1$ | $R^2$ | A | $R^3$ | $R^4$ | $R^5$ | Stereochemie | Substitutionsort der Tetrazolylgruppe | Fp [°C] | Name |
|---|---|---|---|---|---|---|---|---|---|---|
| 2 | $CH_3$ | $CH_3$ | $-CH_2-$ | H | H | $CH_3$ | E | para | 217-218 | (E)-2-(1,1,3,3-Tetramethyl-2,3-dihydro-5(1H)-indenyl)-1-[4-(1H-tetrazol-5-yl)phenyl]-1-propen |
| 3 | $CH_3$ | $CH_3$ | $H_3C-CH$ | H | H | $CH_3$ | E | para | 226-227 | (E)-2-(1,1,2,3,3-Pentamethyl-2,3-dihydro-5(1H)-idenyl)-1-[4-(1H-tetrazol-5-yl)phenyl]-1-propen |
| 4 | $CH_3$ | $CH_3$ | $CH_2$<br>$CH_2$ | H | H | $CH_3$ | Z | para | 217-218 | (Z)-2-(1,1,4,4-Tetramethyl-1,2,3,4-tetrahydronaphth-6-yl)-1-[4-(1H-tetrazol-5-yl)-phenyl]-1-propen |
| 5 | $CH_3$ | $CH_3$ | $CH_2$<br>$CH_2$ | H | $OCH_3$ | $CH_3$ | E | para | 250-251 | (E)-2-(7-Methoxy-1,1,4,4-tetramethyl-1,2,3,4-tetrahydronaphth-6-yl)-1-[4-(1H-tetrazol-5-yl)phenyl]-1-propen |
| 6 | $CH_3$ | $CH_3$ | $CH_2$<br>$CH_2$ | $OCH_3$ | $CH_3$ | H | E | para | 233-234 | (E)-2-(5-Methoxy-1,1,4,4,7-pentamethyl-1,2,3,4-tetrahydronaphth-6-yl)-1-[4-(1H-tetrazol-5-yl)phenyl]ethen |

| Nr. | $R^1$ | $R^2$ | A | $R^3$ | $R^4$ | $R^5$ | Stereo-chemie | Substitutions-ort der Tetra-zolylgruppe | Fp [°C] | Name |
|---|---|---|---|---|---|---|---|---|---|---|
| 7 | $CH_3$ | $CH_3$ | $CH_2$<br>$CH_2$ | H | $CH_3$<br>$-CH_2-CH$<br>$CH_3$ | $CH_3$ | E | para | 239-240 | (E)-2-1,1,4,4-tetramethyl-1,2,3,4-tetrahydronaphth-6-yl)-1-[4-(1H-tetrazol-5-yl)-phenyl]-1-propen |
| 8 | $CH_3$ | $CH_3$ | $CH_2$<br>$CH_2$ | H | H | $CH_3$ | E | meta | 360 | (E)-2-(1,1,4,4-Tetramethyl-1,2,3,4-tetrahydronaphth-6-yl)-1-[3-(1H-tetrazol-5-yl)-phenyl]-1-propen |
| 9 | $CH_3$ | $CH_3$ | $CH_2$<br>$CH_2$ | H | $CH_3$ | $CH_3$ | E | para | 241-242 | (E)-2-(1,1,4,4,7-Pentamethyl-1,2,3,4-tetrahydronaphth-6-yl)-1-[4-(1H-tetrazol-5-yl)-phenyl]-1-propen |
| 10 | $CH_3$ | $CH_3$ | $CH_2$<br>$CH_2$ | H | $n-C_3H_7$ | $CH_3$ | E | para | 251-252 | (E)-2-(7-Propyl-1,1,4,4-Tetramethyl-1,2,3,4-tetrahydronaphth-6-yl)-1-[4-(1H-tetrazol-5-yl)phenyl]-1-propen |
| 11 | $CH_3$ | $CH_3$ | $CH_2$<br>$CH_2$ | H | H | H | E | para | 210 (Zersetzung) | (E)-2-(1,1,4,4-Tetramethyl-1,2,3,4-tetrahydro-naphth-6-yl)-[4-(1H-tetrazol-5-yl)-phenyl]-ethen |

| Nr. | $R^1$ | $R^2$ | A | $R^3$ | $R^4$ | $R^5$ | Stereochemie | Substitutionsort der Tetrazolylgruppe | Fp [°C] | Name |
|---|---|---|---|---|---|---|---|---|---|---|
| 12 | $CH_3$ | $CH_3$ | $CH_2$ $CH_2$ | H | F | $CH_3$ | E | para | 231-232 | (E)-2-(7-Fluoro-1,1,4,4-tetramethyl-1,2,3,4-tetrahydronaphth-6-yl)-1-[4-(1H-tetrazol-5-yl)phenyl]-1-propen |
| 13 | $CH_3$ | $CH_3$ | $CH_2$ $CH_2$ | H | H | $C_2H_5$ | E | para | 160-161 | (E)-2-(1,1,4,4-Tetramethyl-1,2,3,4-tetrahydronaphth-6-yl)-1-[4-(1H-tetrazol-5-yl)phenyl]-1-buten |
| 14 | $CH_3$ | $CH_3$ | $CH_2$ $CH_2$ | H | H | | E | para | 177-178 | 2-(1,1,4,4-Tetramethyl-1,2,3,4-tetrahydronaphth-6-yl)-2-cyclopropyl-1-[4-(1H-tetrazol-5-yl)phenyl]ethen |

## Patentansprüche

1. Vinyltetrazolylphenyl-Derivate der Formel (I)

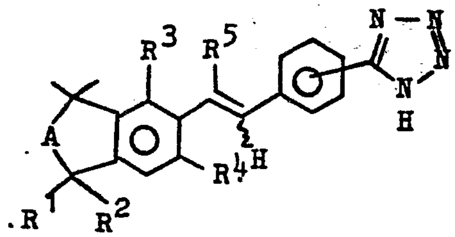

(I),

in der $R^1$ und $R^2$ Wasserstoff oder eine Methylgruppe, $R^3$ und $R^4$ Wasserstoff, Halogen, eine $C_{1-4}$-Alkyl- oder $C_{1-4}$-Alkoxygruppe, $R^5$ Wasserstoff, eine $C_{1-6}$-Alkyl- oder eine $C_{3-6}$-Cycloalkylgruppe und A einen gegebenenfalls durch $C_{1-4}$-Alkylgruppen substituierten Methylen- oder Ethylenrest oder - wenn $R^1$ und $R^2$ Methylgruppen darstellen - auch die Reste $-CH_2-CO-$ oder $-CH_2-\overset{OH}{CH}-$ bedeuten, und ihre physiologisch verträglichen Salze.

2. Vinyltetrazolyl-Derivate der Formel I gemäß Anspruch 1, dadurch gekennzeichnet, daß sie in der E-Form vorliegen und $R^1$ und $R^2$ die angegebenen Bedeutungen besitzen und $R^3$ und $R^4$ Wasserstoff, Methyl und/oder Methoxy und $R^5$ Wasserstoff oder Methyl bedeuten.

3. Verfahren zur Herstellung der Verbindungen der Formel I gemäß Anspruch 1, dadurch gekennzeichnet, daß man ein Azid mit einem Nitril der Formel (II)

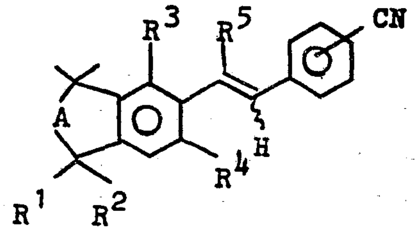

(II),

in der $R^1$, $R^2$, $R^3$, $R^4$, $R^5$ und A die oben angegebenen Bedeutungen haben, umsetzt.

4. Arzneimittel enthaltend eine Verbindung der Formel I gemäß Anspruch 1.

5. Verbindung der Formel I gemäß Anspruch 1 zur Verwendung bei der Bekämpfung von Krankheiten.

Patentansprüche

1. Verfahren zur Herstellung der Vinyltetrazolylphenyl-Derivate der Formel (I)

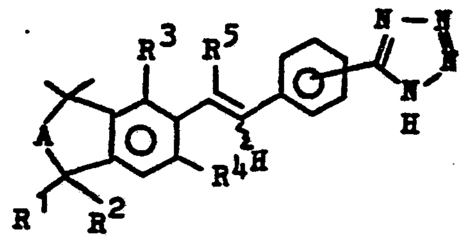

(I),

in der $R^1$ und $R^2$ Wasserstoff oder eine Methylgruppe, $R^3$ und $R^4$ Wasserstoff, Halogen, eine $C_{1-4}$-Alkyl- oder $C_{1-4}$-Alkoxygruppe, $R^5$ Wasserstoff, eine $C_{1-6}$-Alkyl- oder eine $C_{3-6}$-Cycloalkylgruppe und A einen gegebenenfalls durch $C_{1-4}$-Alkylgruppen substituierten Methylen- oder Ethylenrest oder - wenn $R^1$ und $R^2$ Methylgruppen darstellen - auch die Reste $-CH_2-CO-$ oder $-CH_2-CH(OH)-$ bedeuten, und deren physiologisch verträglichen Salze, dadurch gekennzeichnet, daß man ein Azid mit einem Nitril der Formel (II)

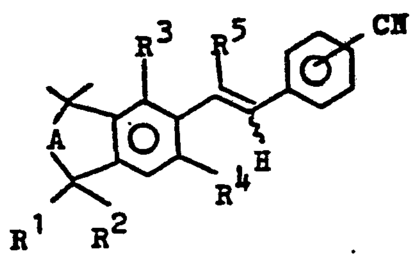

(II),

in der $R^1$, $R^2$, $R^3$, $R^4$, $R^5$ und A die oben angegebenen Bedeutungen haben, umsetzt.

2. Verfahren gemäß Anspuch 1, dadurch gekennzeichnet, daß man Verbindungen herstellt, die in der E-Form vorliegen und worin $R^1$ und $R^2$ die angegebenen Bedeutungen besitzen und $R^3$ und $R^4$ Wasserstoff, Methyl und/oder Methoxy und $R^5$ Wasserstoff oder Methyl bedeuten.